# EUROPEAN PATENT APPLICATION

(11) **EP 3 076 171 A1**
(43) Date of publication of application: **05.10.2016**
(21) Application number: 15161733.9
(22) Date of filing: 30.03.2015
(51) Int. Cl.: G01N 33/00, G01N 1/22, G01N 1/28

(54) **PASSIVE CONTAMINENT SAMPLING DEVICE**

(71) Applicant: Albert-Ludwigs-Universität Freiburg, 79085 Freiburg (DE)
(72) Inventor: Reski, Ralf, D-79254 Oberried (DE); Decker, Eva, D-79104 Freiburg (DE); Beike, Anna, D-70825 Korntal-Münchingen (DE); Giordano, Simonetta, I-80121 Napoli (IT); Adamo, Paola, I-80135 Napoli (IT); Tretiach, Mauro, I-34127 Trieste (IT); Spagnuolo, Valeria, I-80131 Napoli (IT); Bargagli, Roberto, I-53023 Castiglione d'Orcia (IT); Abaol Vinas, Jesus R., ES-Santiago de Compostela (ES); Real Rodriguez, Carlos, ES-27003 Lugo (ES); Fernandez Escribano, Jose, ES-36680 A Estrada (ES); Carballeira Ocana, Alejo, ES-15701 Santiago de Compostela (ES); Lopez Mahia, Purificacion, ES-15002 A Coruna (ES); Muniategui Lorenzo, Soledad N., ES-15004 A Coruna (ES); Prada Rodriguez, Dario, ES-15004 A Coruna (ES); Pineiro Iglesias, Maria, ES-15011 A Coruna (ES); Concha Grana, Estafania, ES-15190 A Coruna (ES); Gonzalez Gonzales, Aridane, ES-35260 Las Palmas (ES); Pokrovsky, Oleg, FR-31290 Gardouch (FR); Rey Asensio, Ana Isabel, ES-32003 Ourense (ES); Ramos Gomez, Julia, ES-15706 Santiago de Compostela A Coruna (ES); Martinez-Abaigar, Javier, ES-26005 Logrono (ES); Zechmeister, Harald Gustav, A-1020 Vienna (AT)
(74) Representative: Dr. Langfinger & Partner

(57) **Abstract**

Passive contaminant sampling device comprising an axenic devitalized moss clone for measuring air pollution.

## Description

The present invention relates to a passive contaminant sensor device comprising an axenic devitalized moss clone.

Atmospheric pollution constitutes one of the most important challenges for the health of next human generations and the environment and in view of this, appropriate monitoring of air pollution is of utmost importance.

For compliance with legal requirements, usually monitoring stations from urban and peri-urban environments are used, but these stations only are really useful when macro-pollutants (e.g. sulphur and nitrogen dioxides and fine and suspended particulate matter (PM)) are assessed in agglomerations. For measurement of other pollutants (i.e. lead, cadmium, arsenic, nickel, mercury and polycyclic aromatic hydrocarbons (PAHs)) there are technical difficulties and their analysis on air is too expensive. As a consequence there is a lack of representative data because these expensive techniques impede their use on an extensive scale.

One of the main problems to the air pollution control is that traditional methods are very expensive. Each fixed complete station costs appr. €120,000 and the annual maintenance appr. €12,000 per year. A respective station provides real-time information for SO₂, NOₓ, CO, O₃, and particulate matter (PM). However, for heavy metals and PAHs, the PM₁₀ has to be collected and subsequently analysed with respect to the levels of metals and PAHs.

The task of monitoring airborne trace element pollution over large areas is arduous, since the concentrations of pollutants are very variable in space and time. In addition, data from automatic devices are accurate but too limited in number to describe spatio-temporal trends of pollutants. Furthermore, automatic devices at monitoring stations can generally detect a limited number of pollutants only.

The use of mosses, liverworts and hornworts (bryophytes) as bioindicators of heavy metal composition in the atmosphere has been known since 1969 when mosses began to be used to monitor the air pollution levels in Europe.

Terrestrial mosses are more frequently used relative to other sorbents because of their lower cost, widespread presence in the environment and high adsorption capacity of pollutants.

One of the most commonly used methods is the "moss-bag technique", developed as described in Goodman GT, Roberts TM. Plants and soils as indicators of metals in the air. Nature (Lond.) 1971;231:287.

Despite the broad application of this technique over past decades, there is still a lack of *i*) standardization of the moss-bag technique and *ii*) the justification of the choice of one particular moss species to be used under variable environmental conditions.

Moss-bags are generally prepared from mosses naturally grown in unpolluted areas so the availability depends on natural and anthropogenic circumstances. Frurhermore, the natural variability on moss elemental composition (including many of the pollutants to be determined) results in variation of the initial conditions of moss-bags over time.

The use of devitalized moss clones as passive contaminant sensors was the topic of an EU-funded collaborative research project (www.mossclone.eu).

Having a standardized biomaterial and exposure procedure, data collected in different world-wide locations could be directly compared for air pollution assessment.

There is thus an ongoing need for new inexpensive and robust tools for monitoring air quality.

It was thus an object of the present invention to provide efficient, inexpensive and readily availpable passive contaminant sensor devices.

This object is achieved with the devices in accordance with claim 1. Preferred embodiments are set forth in the dependent claims and the detailed desription hereinafter.

The term passive contaminant sensor device, as used herein, denotes any device for assembly in any shape or structure comprising a sensor material accessible to air which sensor material is able to detect or accumulate air pollutants.

One example of suitable devices may have the shape and structure of the so called moss-bags. The moss-bag technique initially involves the collection of a moss species from its natural, relatively unpolluted habitat. The moss is than cleaned from extraneous materials (litter, soil..), packed into (nylon net)-bags and suspended in the area of the interest.

However, the passive contaminant sensor devices in accordance with the present invention are not limited in shape or structure to moss-bags but may take any shape or structure suitable and appropriate for the respective monitoring application. The skilled person, based on his professional knowledge, will select the appropriate device form, shape or structure for the intended application.

The passive contaminant sensor devices in accordance with the present invention comprises an axenic devitalized moss clone.

The term axenic, as used herein, describes the state of a culture in which only a single species, variety, or strain of organism is present and entirely free of all other contaminating organisms.

As axenic cultures are derived from very few organisms, or even a single individual, they generally share a relatively narrow gene pool. In the case of an asexual species derived from a single individual, the resulting culture should consist of identical organisms (though processes such as mutation, somaclonal variation and horizontal gene transfer may introduce some degree of variability).

Axenic culturing of mosses is comparatively complicated and a description of a suitable method, for mosses of the *Sphagnum* genera as exemplary moss can be given as follows. It is readily apparent to the skilled person that the amounts of the various chemicals as well as their chemical nature may be modified in accordance with the specific need and are only exemplary here. The skilled person, based on his professional knowledge will be in a position to chose the appropriate conditions and chemicals for the individual application case or the individual moss which is to be cultivated as axenic clone.

Sporangia from a *Sphagnum* species of interest are collected in the field. For spore sterilization, mature capsules are transferred to 600 µL 0.1 % sodium hypochlorite (Merck, Darmstadt, Germany) solution and opened with sterile forceps by squeezing. Sodium hypochlorite solution is prepared freshly and 1 drop of Tween^{®}20 (Merck, Darmstadt, Germany) is added per 10 mL of the solution. After incubation series of 45 s, 1, 1.5, 2, 2.5, 3, 3.5 and 4 min each 75 µL of the mixture is transferred to 4 mL autoclaved water. From this dilution 1 mL is transferred to a sterile Petri dish containing solid Knop medium (1.84 mM KH₂PO₄, 3.35 mM KCl, 1.01 mM MgSO₄ * 7 H₂O, 4.24 mM Ca(NO₃)₂ * 4 H₂O, 45 µM FeSO4 * 7 H₂O) according to Reski and Abel (1985). The Petri dishes are enclosed with Parafilm^{®} (Carl Roth GmbH, Karlsruhe, Germany) and kept under growth conditions of 70 µmol m⁻² s⁻¹ light intensity (Philips TLD 36 W/33/640) and a photoperiod of 16 h light to 8 h dark at 23 °C. After spore germination, single thalloid protonemata are transferred to new Petri dishes containing solid Knop medium. The transfer is done under sterile conditions using needles and a stereo microscope (Stemi 2000-C, Zeiss, Jena, Germany). For sterility control a swap with a needle is done, once on LB medium (10 g/L Bacto-Trypton (Becton, Dickinson and Company, Le Pont de Claix, France), 10 g/L NaCl, 5 g/L Bacto Yeast Extract (Becton, Dickinson and Company, Le Pont de Claix, France)), and once on Knop medium supplemented with 1 % glucose. The sterile controls should be kept for at least 4 weeks at room temperature.

The moss clone used in the devices in accordance with the present invention are devitalized. Devitalization means that the moss is treated to terminate its metabolic activities, i.e. there will be no changes after devitalization caused by the metabolism of the moss. Thus the chemical composition of the moss and its properties should basically freeze after devitalization.

Devitalization provides a number of advantages. First, it enables to keep constant over time the efficiency of the capture of the contaminants to be determined. The uptake of contaminants is a passive process mainly caused by adsorption phenomena and is thus independent of the vitality of the moss. The same applies to the cation exchange capacity and the capacity for particle retention; these properties are again independent of the vitality of the moss.

The completion of devitatilization can be monitored through metabolic ativities; once they have stopped, the devitalization can be deemed to be complete.

The method to achieve the devitalization are not particularyly critical and the skilled person is aware of suitable process conditions for achieving the desired result. Nevertheless, the conditions of devitalization have to be properly protocolled to ensure reliability and reproducability.

There are several methods for devitalization available and known to the skilled person.

Acid washing is one common method in this regard which includes immersion of the selected moss matreial in an acid medium with the aim of leaching metal ions from the cell wall and disrupting cell walls thereby stopping the prerequisites for a functioning metabolism of the moss.

The results of the acid treatment are determined by various parameters: (i) number of washes; (ii) duration of washing; (iii) inclusion (or not) of shaking; (iv) acid used and concentration, and (v) ratio between the weight of the moss and the volume of the acid solution.

Without being limited to such specific process it may be said that the most common acid washing step comprises one washing step the duration of which may vary over a broad range. Generally, washing times of between 0.5 and 96 hours may be used in many cases.

Suitable acids may be selected from a broad range of acids but in some cases HNO₃, at concentrations varying between 0.025 and 1 mol/l and HCl at concentrations between 0.01 and 0.5mol/l may be mentioned by way of example.

Furthermore, the amount of acid used, based on the weight of the moss treated should be registered to ensure reproducibility.

Another common method for devitalization is a heat treatment or oven drying. It comprises maintaining the moss usually in an oven at a high temperature for periods of time usually up to 24 hours. The temperature may also be gradually increased over time in accordance with a certain temperature profile or certain treatment steps at different temperatures may be applied. Treatment times of up to 48 h at temperatures of up to 120 °C may be used. It is also possible to apply several treatment steps at different temperatures consecutively.

Heat treatment has some advantages over acid washing: it is eco-friendly and it maintains with better certainty the particular morphology and leaflet arrangement of the moss without altering the capacity for capture of the contaminant. The acid treatment deteriorates the morphology to a greater extent as it causes a rupture of the tissues.

The disadvantage in some mosses is that the shoots become brittle and the small leaf fragment fall off the stem.

Devitalization by drying does not release metals bound to cationic exchange sites which might negatively influence the capacity for the uptake. This may be overcome by the use of chelating agents such as EDTA, penicilline or dimercaprol prior to washing with water.

In some cases it has been found that the surface texture after acid washing or heat treatment is very similar. Given that the surface interception of air-borne particulates is likely to play the major role for the accumulation of the contaminants.

In order to assure reproducability, the devitalization process should be properly monitored and laid down precisely in a suitable document.

Whereas there is no specific limitation as to the type of moss, a number of mosses have proved to be particularly suitable for the present invention.

Accordingly, moss clones obtained from *Sphagnum* mosses (*Sphagnum* sp.), sheet mosses (*Hypnum* sp.), *Pseudoscleropodium purum* or *Brachythecium rutabulum,* in particular from *Sphagnum* mosses, and particularly preferred from *Sphagnum palustre.*

*Sphagnum* is a genus of approximately 120 species, which are also commonly known as peat moss. Individual peat moss plants consist of a main stem, with tightly arranged clusters of branch fascicles usually consisting of two or three spreading branches and two to four hanging branches. The top of the plant, or capitulum, has compact clusters of young branches. Along the stem are scattered leaves of various shapes, named stem leaves; the shape varies according to species. The leaves consist of two kinds of cells; small, green, living cells (chlorophyllose cells, chlorocysts), and large, clear, structural, dead cells (hyaline cells, hyalocysts).

In many cases it has been found to be advantageous if the specific surface area of the clone used in the devices in accordance with the present invention, exceeds the specific surface area of the moss form which the clone was obtained, by at least 50 %, preferably at least 70 % and even more preferably at least 80 %. To verify this parameter, the specific surface area of the moss before cloning is measured and after cloning the respective measurement is carried out on the clone thereby yielding two values, the ratio of which can then be determined.

For the purpose of this inventions, the specific surface area is determined using the so called BET method (Brunauer, Emmett and Teller) using the N2 multipoint adsorption technique using a Quantachrome Autosorb Automated Gas Sorption System with 10 to 14 adsorption points after 24 hours of degassing at 120°C. The details of the instrumentation setup are described in the Quantachrome manual to which reference is made here for further details.

Since the parameter of interest is a relative value, namely the ratio of two values determined with different substrates, it is important to use exactly the same method for measuring both substrates. Differences between the two measurements can lead to false results.

Generally, however, the ratio of two surface areas determined under identical conditions has not a high degree of variation depending on the specific measurement method. In case of doubt, whether the specific surface area ratio of the clone is at least 50 % higher than that of the moss from the field, the reference method to be used is the BET method described and defined above and in the manual of the equipment supplier.

In accordance with another preferred embodiment of the present invention, the axenic devitalized clone in the devices of the present invention has an Al-content, based on dry weight, which is at least 60 %, preferably at least 70 % and most preferably at least 85 % less than the Al content of the natural moss from which the clone was obtained.

Furthermore, in accordance with another preferred embodiment, the Fe-content of the axenic devitalized clone in the devices of the present invention, based on dry weight, is at least 60 %, preferably at least 70 % and most preferably at least 75 % below the Fe content of the natural moss from which the clone was obtained.

Again, to achieve proper and reproducible results it is essential that the determination of the metal content is made for both substrates under identical conditions.

Suitable methods for determining Al-and Fe-content are known to the skilled person so that no further details need to be given here.

The elementary composition is one of the key factors controlling the adsorption capacity of the clones, notably at low metal concentration. The trace metal composition of clones helps to standardize the conditions for using them as bioaccumulators in the devices in accordance with the present invention. The elementary composition of clones reflects the composition of the culture medium because the strains (diferernt to natural moss) were not exposed to any atmospheric pollution.

Preferably, the clones used in accordance with the present invention have a content of at least one of the elements derived from Na, Mg, Ca, Ti, V, Mn, Co, Ni, Cu, Zn, Ga, Ge, A, Rb, Sr, Zr, Nb, Ag, Cd, Cs, Ba, La, Ce, Pr, Nd, Sm, Eu, Gd, Dy and Ho which is at least 50 % preferably at least 60% and even more preferably at least 70 % below the content of the respective element in the natural moss from which the clone was obtained.

The metal content of mosses from the field in many cases reflects metal deposition from the atmosphere in their original location. As mentioned above, the elementary composition of clones in contrast reflects mainly the composition of the culture medium because the strains were not exposed to any atmospheric pollution during cloning in the bioreactor.

Elementary composition of mosses can be measured by ICP-MS after full acid digestion in a microwave. Mineralization solution without moss samples (i.e. blanks) may be used as negative controls to ensure no contamination from the digestion acid. Due to the cloning technology there are in most cases practically no significant differences in metal concentration between different clone lines, neither between different production sites.

In biomonitoring studies using moss clones, homogeneity and magnitude of background or reference levels of trace metals are very important issues to assess the degree of atmospheric contamination. Bioaccumulation of airborne trace elements is assessed by the difference between post-exposure and pre-exposure moss content and thus the detection accuracy and the limit of detection are improvend with lower contents of the trace elements in the clone.

Furthermore, for obvious reasons, the lowest the reference contents in pre-exposure samples, the highest is the ability of the moss clone to detect pollution inputs.

Experiments have shown that in many cases axenic devitalized clones of *Sphagnum palustre* have the lowest content of a significant number of trace elements and for this reasn *Sphagnum palustre* clones are preferred in accordance with the present invention.

Polycyclic aromatic hydrocarbons (PAH) are a family of chemical compounds composed of carbon and hydrogen atoms which form at least two condensed aromatic rings. PAH originate from fossil or non-fossil fuels by pyrolysis or pyrosynthesis. They are emitted into the atmosphere mainly from anthropogenic sources but they also originate from natural ones such as volcanic eruptions and forest fires. The main sources of PAHs in the environment are aluminum production, coke production from coal, wood preservation and fossil fuel combustion (traffic, domestic heating, and electricity production). PAHs are considered as persistent organic pollutants (POPs) due to their low rates of degradation, toxicity and potential for both long-range transport and bioaccumulation in living organisms. Regulation of PAH emissions and reliable monitoring of PAH concentration in ambient air is thus of paramount importance for public health.

Investigations have shown that the devices in accordance with the present invention comprising axenic devitalized moss clones are particularly suited as passive sampler for PAH analysis, since this allows the standardized determintion of PAH and with initial concentrations of PAHs lower than the natural moss, which allows its use for the monitoring of unpolluted areas, which is in many cases not possible with natural moss as the content of PAH in the moss is already higher than the content in the atmosphere under investigation.

The PAH regularly analyzed include in terms of volatility light PAHs (naphthalene, acenaphthylene, acenaphthene, fluorene), intermediate PAHs (phenanthrene, anthracene, fluoranthene, pyrene) and heavy PAHs (benzo(a)anthracene, chrysene, benzo(b)fluoranthene, benzo(j)fluoranthene, benzo(k)fluoranthene, benzo(e)pyrene, benzo(a)pyrene, dibenzo(a,h)anthracene, benzo(ghi)perylene and indene(1,2,3-cd)pyrene).

The devices in accordance with the present invention are also suitable for the sensing and monitoring of metals, in particular heavy metals, and their compounds.

Heavy metals, a term applied to a large group of metals and metalloids often defined "trace elements" due to their low natural background concentrations in the environment, are one of the main air pollutants. They derive from both natural and anthropogenic sources (e.g. smelting, refining, metal production, plating and mining activities, manufacturing processes, vehicle emissions, waste incineration) and they are persistent, since they cannot be degraded or destroyed. Once emitted into the atmosphere, heavy metals could be easily transported far from sources as airborne contaminants before depositing in soil and waters, so as to represent a serious toxicity factor for all the living organisms, especially by biomagnification and bioaccumulation in food-chains. The heavy metal toxicity in humans is often metal-specific and related to the exposure conditions. Some elements (i.e. Cu, Fe, Mn and Zn) are essential for human metabolism but they may cause acute or chronic negative effects also at very low concentrations.

The devices in accordance with the present invention have proved to be useful in particular also for long term monitoring, i.e. monitoring very variable concentration of airborne trace elements on vast areas and for long times.

Standard physico-chemical methods, although they can provide relatively quickly precise and accurate data, are expensive and limited to few pollutants and not useful for retrospective surveys on environmental pollution.

To the contrary, the air quality monitoring with the devices in accordance with the present invention using moss clones for collecting the pollutants provide a good alternative to acquire data, as it is a relatively rapid, eco-friendly and low-cost method that can provide qualitatively and quantitatively accurate and reliable pollution data, representative of the territory.

As has been outlined before, the devices in accordance with the present invention are not subject to specific limitations as far as structure and form or shape is concerned.

Without being limited thereto, a device type commonly known as moss bag will be described in more detail now as such bags are suitable to take up the axenic moss clones used in accordance with the present invention.

The moss-bag technique was introduced to overcome one of the most frequent problems encountered in biomonitoring surveys, i.e. the lack of indigenous moss material, especially in areas with unsuited environmental conditions (e.g. because there is a strong air pollution).

Basically, moss bags comprise mosses as material capturing the air pollutants which are the subject of the monitoring in a suitable container format which on one hand protects the moss from being destroyed, leached out or blown away on a suitable carrier which may be in the fom of a net with a certain mesh size allowing the pollutant molecules to pass through an retaining greater particles which could occupy the moss surface too quickly and thereby negatively influence the capability of the moss to accumulate the pollutants. The accumulation kinetics depend on the density of thematerial inside the bag, the ratio between the external surface and the mass of the material and the surface exposed for the interception of rain.

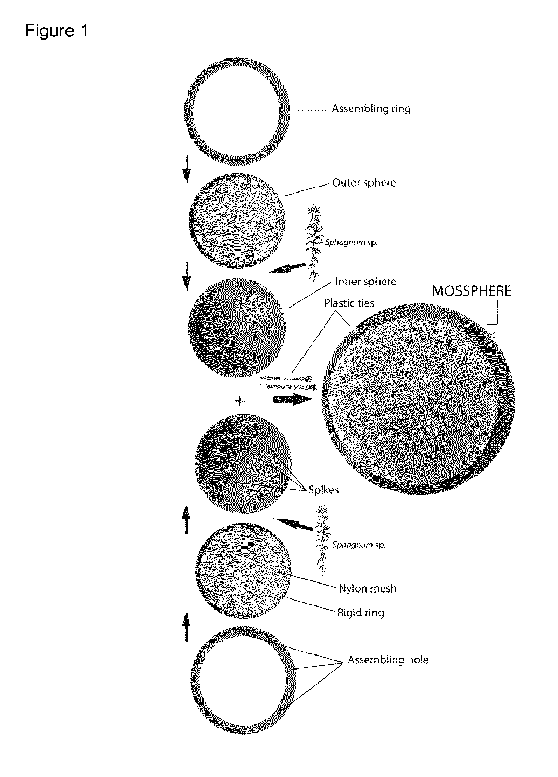

In a series of experiments, a certain device form (hereinafter referred to as mosssphere) has been found to be particularly suitable for many purposes, which is now described with reference to Fig. 1 in more detail. It is readily apparent that the materials of the various parts and the dimesnions can be modified in accordance with the needs of the specific application and the skilled person will do so using his professional experience.

The moss exposure device has a spherical shape as being the best way to expose the moss as it is aerodynamic, independent of the wind direction and the geometrical shape has the greater volumetric content.

The device is primarily made of an inert plastic (e.g. polypropylene, PP) and is formed by two perforated hollow spheres, one placed inside the other (Figure 1). Both spheres are made up of two hemispheres that are assembled with two plastic rings and ties.

The device is composed of 6 pieces; 2 inner and 2 outer hemispheres, and 2 connecting rings. The outer size of the sphere has a diameter of 13 cm. This distance increases to 14 cm when the ring width connecting the two hemispheres is included. Both hemispheres are surrounded by a ring of rigid plastic for easy assembling.

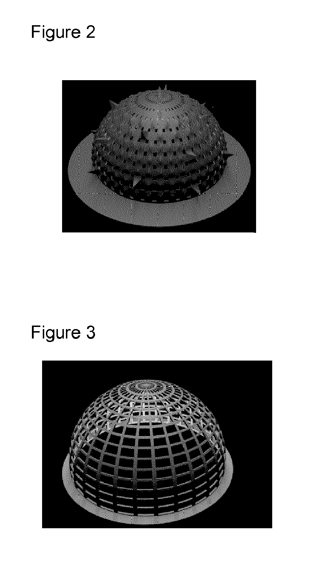

The inner sphere is rigid (e.g. polypropylene, PP), smaller (95 mm sphere diameter + 16 mm outer ring) and has spikes (6 mm high x 3 mm wide x 4 mm deep) on the outside that are specifically designed to hold and spread the moss homogeneously through the whole surface (Figure 2). This sphere has also holes of variable size and spines (smaller than the previously mentioned but used for the same function). (Figure 2). The perforations are designed to promote the passage of air through the mosssphere.

The outer sphere has a larger size (13 cm ring diameter + 1 cm) and has a 2 mm nylon mesh surrounded by a ring of rigid plastic (e.g. PP) (Figure 3).

The two connecting rings have a 13 and 14 cm of inner and outer diameter respectively. Therefore the width of the circumference is 10 mm. The ring has a depth of 2 mm and the width is 10 mm, of which 5 mm of the inner side have a cleft of about 1.2 mm where the outer rings of semispheres are inserted. Both rings have four equally spaced holes on the outer edge.

Cloned moss (the axenic devitalized moss clone) is introduced between the two hemispheres (internal and external) of the mosssphere (Figure 1), then, the two hemispheres are joined together with the external ring and four small plastic ties which are introduced into the holes of the two outer rings.

The following experimental data show the usability of the devices in accordance with the present invention comprising an axenic, devitalized moss clone of *Sphagnum palustre* for sensing and monitoring a wide range of air pollutants. The specific clone described hereinafter just serves as an example of a clone which has been found advantagoeus in certain cases but is is readily apparent to the skilled person that in principle other clones obtained from other mosses or with different methods could also be useful,

### Example 1 - Preparation of axenic moss clone

The axenic moss clone from *Sphagnum palustre* used for the experiments was obtained as follows (following Beike et al. Plant Cell Tiss Organ Cult (2015) 120:1037-1049).

Sporangia from five *Sphagnum* species were collected in the field. For spore sterilization, mature capsules were transferred to 600 µL 0.1 % sodium hypochlorite (Merck, Darmstadt, Germany) solution and opened with sterile forceps by squeezing. Sodium hypochlorite solution was prepared freshly and 1 drop of Tween^{®}20 (Merck, Darmstadt, Germany) was added per 10 mL of the solution. After incubation series of 45 s, 1, 1.5, 2, 2.5, 3, 3.5 and 4 min each 75 µL of the mixture were transferred to 4 mL autoclaved water. From this dilution 1 mL was transferred to a sterile Petri dish containing solid Knop medium (1.84 mM KH2PO4, 3.35 mM KCl, 1.01 mM MgSO4 * 7 H₂0, 4.24 mM Ca(NO3)2 * 4 H2O, 45 µM FeSO4 * 7 H2O) according to Reski and Abel (1985). The Petri dishes were enclosed with Parafilm^{®} (Carl Roth GmbH, Karlsruhe, Germany) and kept under growth conditions of 70 µmol m⁻² s⁻¹ light intensity (Philips TLD 36 W/33-640) and a photoperiod of 16 h light to 8 h dark at 23 °C. After spore germination, single thalloid protonemata were transferred to new Petri dishes containing solid Knop medium. The transfer was done under sterile conditions using needles and a stereo microscope (Stemi 2000-C, Zeiss, Jena, Germany). For sterility control a swap with a needle was done, once on LB medium (10 g/L Bacto-Trypton (Becton, Dickinson and Company, Le Pont de Claix, France), 10 g/L NaCl, 5 g/L Bacto Yeast Extract (Becton, Dickinson and Company, Le Pont de Claix, France)), and once on Knop medium supplemented with 1 % glucose. The sterile controls were kept for at least 4 weeks at room temperature.

For cultivation of single clones of *S. palustre* on solid medium, gametophores that developed from thalloid protonema were transferred to solid Knop medium supplemented with microelements (50 µM H₃BO₃, 50 µM MnSO₄ 1 H₂O, 15 µM ZnSO₄ 7 H₂O, 2.5 µM KI, 500 nM Na₂MoO₄ 2 H₂O, 50 nM CuSO₄ 5 H₂O, 50 nM Co(NO₃)₂ 6 H₂O). The Petri dishes were enclosed either with Parafilm^{®} only (Carl Roth GmbH, Karlsruhe, Germany) or with micropore^{™} (VWR International GmbH, Darmstadt, Germany) covered with Parafilm^{®}.

For cultivation of *S. palustre* clones in liquid medium, gametophores were transferred to Erlenmeyer flasks filled with 50 or 200 mL liquid medium, respectively, or to aerated round-bottom flasks containing 5 L liquid medium. For standard cultivation in flasks, liquid Knop medium supplemented with microelements (ME), 0.3 % sucrose and 1.25 mM ammonium nitrate (NH₄NO₃) was used. For large scale production in bioreactors, sucrose concentration was increased to 2 %.

The pH of the medium was adjusted to 4.8 with KOH and HCl before autoclaving. Ammonium nitrate solution was sterile filtered and added after autoclaving. The pH was measured using a pH electrode (pH 197-S, WTW GmbH, Weilheim, Germany). After autoclaving, a previously adjusted pH of 4.8 decreased to 4.1 (±0.1, n = 3), while a pH of 2.8 stayed at 2.8 (±0.03, n = 3), a pH of 3.8 decreased to 3.6 (±0.03, n = 3) and a pH of 5.8 decreased to 5.2 (±0.2, n = 3). In the following, the pH before autoclaving is described.

After transfer of *S. palustre* gametophores to liquid medium, the flasks were enclosed with silicone sponge closures (Hirschmann, Eberstadt, Germany). The suspension cultures were shaken continuously at 120 rpm on a shaker (B. Braun Biotech International, Melsungen, Ger-many) in a climate chamber. Changes in pH were monitored during cultivation with a pH electrode starting from ten small gametophores (<0.5 cm) in 50 mL growth medium in flasks.

For cultivation of *S. palustre* in the bioreactor, photo-bioreactors with 5 and 12 L working volume were used (Applikon, Schiedam, The Netherlands). For the 5 L bioreactors, the light intensity was set to 120 µmol m⁻² s⁻¹ using light tubes (Philips TLD 18 W/25) at a photoperiod of 16 h light to 8 h dark. The 12 L bioreactors were illuminated with continuous light at 210 µmol m⁻² s⁻¹ with LED tubes. An adjustment of the pH was achieved by automatic titration with 0.5 M KOH and 0.5 M HCl. If the pH was not adjusted continuously, it was tracked during cultivation with an internal pH electrode. The bioreactors were aerated with 0.3 vvm air. The medium was used as described above, however for large scale production 2 % sucrose instead of 0.3 % sucrose were added. Before inoculating the moss in the bioreactors fresh weight was determined in laminar flow benches (AV-100, Telstar, Spain or Holten, Lami-nair, Thermo Scientific, Dreieich, Germany) with a scale (B502-S, Mettler Toledo, Spain or L 610 D, Sartorius, Gottingen, Germany) using a glass beaker (Simax, Säz-ava, Czech Republic) with a plastic filter or a Steritop^{®} filter (Millipore Corporation, Billerica, MA, USA) with a vacuum pump (Vacuubrand MZ 2C, Vacuubrand GmbH and Co, Wertheim, Germany).

In order to test whether *S. palustre* growth can be enhanced by regular disruption with an Ultraturrax (Ika, Staufen, Germany), gametophores were disrupted at 4,000-18,000 rpm for 10 s up to 1 min. As this sub-cultivation technique used for example for vegetative propagation of the moss *Physcomitrella patens* (Grimsley et al. 1977) was not applicable for *S. palustre* gametophores, the peat moss cultures were disrupted manually using forceps or an autoclavable bottle (17 cm 9 7 cm, Nalgene™, Thermo Scientific, Dreieich, Germany) with inert metal chicanes, i.e. screws STS-plus KN6041 5 9 30-T25 (Schriever, Lüdenscheid, Germany) by shaking the culture for 1 min within the device.

For analyzing the effects of previous disruption and inoculum density on the biomass yield, comparative cultures of each two times 1, 5 and 8 g fresh weight (FW) were started in flasks containing 200 mL liquid Knop medium supplemented with ME, 0.3 % sucrose and 1.25 mM NH₄NO₃ (pH 4.8). One of both cultures was disrupted before cultivation by shaking the gametophores for 1 min within the device, while the other one was not disrupted. After 2 and 4 weeks of cultivation the FW was determined using a scale (L 610 D, Sartorius, Göttingen, Germany). Before weighing, the gametophores were filtered for 1 min using a Steritop^{®} filter (Millipore Corporation, Billerica, MA, USA) and a vacuum pump (Vacuubrand MZ 2C, Vacuubrand GmbH and Co, Wertheim, Germany).

Specific surface area (SSA) of whole moss samples was measured using B.E.T. N2 multipoint adsorption technique using Quantachrome Autosorb Automated Gas Sorption System, with 10 to 14 adsorption points, after 24 h of degasing at 120°C. The typical uncertainty of these measurements was 10%.

SSA results indicate that clones had higher surface area (clone-2a = 24 ± 1 m² g⁻¹; clone-12a = 28 ± 1 m² g⁻¹) than the samples from the field (10.8 ± 0.3 m² g⁻¹). The correlation coefficient was always higher than 0.99 and the differences between clones and natural samples were well beyond the analytical uncertainty.

The surface acid-base titration carried out at pH of 3 to 10 in triplicates for 120°C-inactivated naturally grown *Sphagnum sp.* and *Sphagnum palustre* clones demonstrated that the pH value corresponding to zero net proton adsorption (pH_{PZC}) was equal to 4.5 ± 0.1 for moss from nature, which was significantly lower than that for clones (5.65 ± 0.05 and 5.60 ± 0.06 for lines 2a and 12a , respectively). The excess of charge was similar for both clones, and the magnitude of the surface charge of clones was significantly higher than that of *Sphagnum sp.* from the field.

The pKa values suggested the presence of five possible functional groups: carboxyl/phosphodiester, carboxyl, phosphoryl, amine and polyphenols with total proton-binding sites of 0.648 mmol g-1 (field sample), 1.564 mmol g-1 (clone-2a) and 1.309 mmol g-1 (clone-12a).

The relative amount of various tentative functional groups was different between the studied mosses. Clones-2a and 12a had 1.04 and 0.742 mmol g-1 of carboxyl/phosphodiester (pKa = 3.3 - 5.8), which was a factor of 2 to 3 higher than that for field sample, having only 0.355 mmol g-1. In terms of phosphoryl groups, clone-12a exhibited the highest value (0.817 mmol g-1), one order of magnitude than that of clone-2a (0.079 mmol g-1) and the field sample (0.043 mmol g-1). Clones also exhibited a factor of 1.5 higher concentration of surface amines (pKa = 8.05 - 9.35) and polyphenols (pKa ~ 10.30).

Consequently, the moss from the field demonstrated a factor of 1.5 to 2 lower mass-normalized binding site density compared to clones and thus their metal adsorption capacities should be also lower than those of clones.

Elementary composition of mosses was measured by ICP-MS after full acid digestion in a microwave. Mineralization solution without moss samples (i.e. blanks) were used as negative controls (1 blank every 10 samples) to ensure no contamination from digestion acid. M2 and M3 certified reference mosses, obtained from the Finnish Forest Research Institute, were used as positive controls. Deionized water was used to prepare all samples, blanks and standard solutions.

The elementary composition of a *S. palustre* clone reflects the composition of the culture medium because the strains were not exposed to any atmospheric pollution. Among *~* 40 major and trace elements, only P and Mo (used to regulate the pH for media preparation and necessary for cellular metabolism) were slightly higher in the clones compared to the field sample. The latter was enriched in all other major and trace elements reflecting metal deposition from the atmosphere in its original location. Thus, *Sphagnum* moss from the field contained 3.55 mg kg-1 and 36.0 mg kg-1 of Cu and Zn, respectively, which is a factor of 2 to 4 higher than both clones. The other major and trace metals were a factor of 2 to > 10 lower in laboratory clones compared to naturally grown *Sphagnum* moss. This impoverishment of laboratory samples was especially remarkable for Al (x 300), V (x 400), Ga (x 100), As (x 500), Cd (> 1000), REEs (> 1000) and Pb (x 400).

According to the statistical analysis, there were practically no significant differences on metal concentration between different clone lines, neither between different production laboratories. A further significant reduction in element content occurs when a moss clone is EDTA washed [20' with 10 mM EDTA (1 L EDTA/12.5 g d.w. moss) and 20' (x 3 times) with distilled water (1 L distilled water/10 g d.w.)] and devitalized (3 consecutive oven drying cycles of 8 h each at 50, 80 and 100 °C) prior to be exposed in bags.

### Example 2 - Use of the axenic moss clones obtained in accordance with Example 1 for PAH monitoring

PAH-Mix 45 (10 µg ml⁻¹ in cyclohexane containing acenaphthene, acenaphthylene, anthracene, benz(a)anthracene, benzo(b)fluoranthene, benzo(k)fluoranthene, benzo(g,h,i)perylene, benzo(a)pyrene, benzo(e)pyrene, chrysene, dibenz(a,h)anthracene, fluoranthene, fluorine, indene(1,2,3,c,d)pyrene, naphthalene, perylene, phenanthrene and pyrene was supplied by Dr. Ehrenstorfer GmbH (Augsburg, Germany). Individual standards of benzo(j)fluorathene (10 µg ml⁻¹ in cyclohexane), retene (10 µg ml⁻¹ in cyclohexane), [²H₁₂] chrysene (chrysene-d₁₂, 10 µg ml⁻¹ in cyclohexane) (surrogate), [²H₁₄] dibenz[a,h]anthacene (dibenz[a,h]anthacene-d₁₄, 10 µg ml⁻¹ in cyclohexane) and [²H₁₀] anthracene (anthracene-d₁₀, 100µg ml⁻¹ in cyclohexane) were supplied by Dr. Ehrenstorfer GmbH (Augsburg, Germany). D-labelled PAH surrogate Cocktail 200 µg ml⁻¹ in 50% methylene chloride (D₂, 99.9%) and 50% methanol (D₂, 99%) containing [²H₈] acenaphthylene (acenaphthylene-d₈), [²H₁₂] benzo[a]pyrene (benzo[a]pyrene-d₁₂), [²H₁₂] benzo[g,h,i]perylene (benzo[g,h,i]perylene-d₁₂), [²H₁₀] fluoranthene (fluoranthene-d₁₀), [²H₈] naphthalene (naphthalene -d₈), [²H₁₀] phenanthrene (phenanthrene-d₁₀), and [²H₁₀] pyrene (pyrene-d₁₀) was supplied by Cambridge Isotope Laboratories, Inc. (Andover, MA, USA).

The working standards were prepared as follows: 0.5 µg ml⁻¹ of parent PAHs in hexane, 0.2 µg ml⁻¹ of surrogate labelled standards (surrogate cocktail + chrysene-d₁₂) in hexane, 0.5 µg ml⁻¹ of internal standard (anthracene-d₁₀) in hexane.

Dichloromethane (DCM) Super Purity grade was supplied by Romil (Cambridge, UK), hexane (H) Unisolv^{®}, for organic trace analysis, was purchased from Merk (Darmstadt, Germany).

Sorbents used for the dispersion were: diatomaceous earth acid washed not further calcined and silica neutral supplied by Sigma (Sigma-Aldrich Chemie Gmbh, Germany), octadecyl functionalized silica Supelclean-Envi 18 (Supelco, Bellefonte, USA), and Sea sand pro analysi (Merk). All the sorbents were washed with H and DCM:H (20:80) mixture before using. Silica was activated a 135°C, 12h, and then partially deactivated with milli-Q water. Clean-up was performed using Envi-Florisil SPE glass tube (1 g) supplied by Supelco.

### Sampling

The moss clone sample obtained in the bioreactor in accordance with claim 1 was washed with 10 mM ethylendiaminetetracetic acid (EDTA) (using 1 L for each 12.5 g d.w. of moss) and 3 times with pure water (1 L each 10 g d.w.). The devitalization was performed by oven: 8h at 50°C, 8h at 80°C and 8h at 100°C.

Devitalized moss samples were exposed for 90 days in five different locations: an urban site, in a city (ca. 250,000 inhabitants) in a point affected by road traffic and close to the marine coast; a sub-urban site in an open green area, at 5 km far from the previously mentioned city and also close to the sea (ca. 500 m); an industrial site at 1 km far from a metallurgical industry in an industrial area, close to a thermal-power station; an agricultural site, in an area with meadows, crop and pasture fields; and a background site in a remote area selected as background atmospheric contamination point by the EMEP-VAG-CAMP Spanish Network for atmospheric pollution control.

The sampling for total deposition was carried out according to EN 15980:2011 "Determination of the deposition of benzo(a)anthracene, benzo(b)fluoranthene, benzo(j)fluoranthene, benzo(k)fluoranthene, benzo(a)pirene and dibenzo(a,h)anthracene and indene(1,2,3-cd)pyrene". To carry out the sampling a global collector is used which consist of a cylindrical funnel and a glass bottle, both must be made of glass. The vertical section of the funnel must be at least as higher as its diameter and the area of the cross section of the funnel and the bottle size should be adjusted to the expected amount of rainwater during the sampling period. The collector was open to the atmosphere and sampling during 6 weeks.

The sampling for suspended particulate matter was performed following the standardized method which has been approved in 2008: "EN 15549: Air quality. Standard method for the measurement of the concentration of benzo(a)pyrene in ambient air." The concentrations of pollutants should be referred to PM₁₀ which collection is described in "EN 12341:1999: Air quality. Determination of the PM₁₀ fraction of suspended particulate matter. Reference method and field test procedure to demonstrate reference equivalence of measurement methods."

The sampling was carried out by a high/medium/low volume samplers and each filter was used for each sampling day (24 hours) during 6 weeks. The volume sampler has a single stage impactor designed to achieve its cut-off for 10 µm at a determined flow rate. Quartz filters are used to collect the PM₁₀ daily, and a pool sample was analysed

After the exposure, the samples were collected, oven-dried (24h at 40°C) and then ground in an ultracentrifuge mill (RetschZM100). For the optimization, an unexposed moss clone sample was used.

### Extraction and clean up

0.25 g of moss sample (with 50 µl of working solution of surrogates) was blended with 0.5 g of C₁₈ in an agate mortar, during 3 minutes. Then the homogeneous dispersion was deposited in the top of a Florisil SPE tube (1 g) with 0.5 g of anhydrous sodium sulphate, all previously washed with 4 ml H and 4 ml dichloromethane (DCM):hexane (H) (20:80).

The PAHs were eluted with 10 ml of H and 10 ml of DCM:H (20:80) mixture using a Visiprep vacuum distribution manifold from Supelco (Bellefonte, PA, USA). The eluate was concentrated to approximately 0.3 ml in a Syncore® Analyst evaporator from Büchi Labortechnik AG (Flawil, Switzerland) and then 20 µl of internal standard was added. All was transferred to a vial and analysed by PTV-GC-MS-MS for PAH determination.

### Gas chromatography-tandem mass spectrometry

Gas chromatography was performed with a Thermo-Finnigan (Waltham, MA, USA) Trace GC chromatograph equipped with a GC PAL autosampler (CTC-Analytics, AG, Switzerland), PTV injector, and coupled to an ion trap mass spectrometer (Polaris Q). Xcalibur was the data processor. The system was operated in electron impact mode (EI; 70 eV) and tandem mass spectrometry was the detection mode. The parent and product ions and retention time of PAHs studied are shown in Table 1. The compounds in the table are separated by groups, with the labelled surrogate used for the quantitation of each group in italics. Transfer line temperature was set at 300°C and ion source temperature at 250°C. Helium (99.9999%) was used as the collision gas at the ion trap chamber, and as carrier gas, under constant flow rate of 1 ml min⁻¹.

The separation was achieved with a DB-XLB column (60 m x 0.25 mm, 0.25 µm film thickness) (J&W Scientific, Folsom, CA, USA). The GC oven temperature program used was: 50°C (3 min), increased by 4°C min⁻¹ to 325°C (held for 20 min).

A Silcosteel® liner, with 2 mm of inner diameter, glass wool packing for programmed temperature vaporisation (PTV) was purchased from Thermo Finnigan (Thermo Electron Corporation, Waltham, USA). The injected volume was 25 µl, the injector programme was started at 55°C and up at 3°C s⁻¹ until 300°C (held for 20 min).

Instrumental and procedural blanks were systematically evaluated (a blank for each batch) and maintained at minimum. Two standard solutions containing all target PAHs were injected for each batch for chromatographic control. Dibenz[a,h]anthracene-d₁₄ (to correct dibenz(a,h)anthracene, indene(1,2,3-cd)pyrene, benzo(ghi)perylene-d₁₂ and benzo(ghi)perylene) and anthracene-d₁₀ (for the other PAHs) were used as internal standards (20 µl of 0.5 µg ml⁻¹ was added to the final eluate). Quality control of the complete procedure and quantification were performed using labelled PAHs as surrogates.

### Results

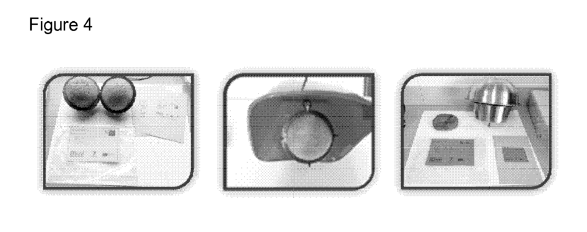

Firstly, it was carried out a comparison between different devices in which moss clone was introduced to perform passive sampling of PAH. The three devices are shown in Figure 4 from left to right. The first device was a mosssphere as described before with reference to Figures 1 to 3 which was filled with 3 g of the moss clone. The second device was a sheltered Mosssphere (also filled with 3 g of moss clone) where the device was sheltered by a surrounding protective shelter reducing the influence of wind and rain. and the last device was a sheltered moss clone "bell" as shown to the right in Fig. 4.

In those sampling sites where it is is probably to find PAH associated to airborne particulates (contaminated areas), it was observed that the PAH concentrations found in the Moss clone are higher for the intermediate to heavy PAH, mainly associated to particulate matter (it is not usual to find these compounds in gas phase under the meteorological conditions of the studied areas).

In the case of less polluted areas, like rural and rural background sites, no differences between the 3 types of devices were found. Probably, this was due to fewer concentration of PAH associated to particulate matter.

Moss clone "Mosssphere" shows higher concentrations of PAHs than sheltered moss clone and this one than sheltered moss clone "bell". From these experiments it could be concluded that the sheltered devices hampered the uptake of particulate matter on the moss, which is significant in the case of the most polluted areas. Moreover, no effect of the washout of the rain in the unsheltered devices was found.

A comparison of the PAH in the first device and in the total deposition, showed a very similar distribution of the PAHs (light, intermediate and heavy) in the moss and the total deposition samples. Moreover, when the distribution profile of both sampling devices was compared it could be noticed that the profiles were very coincident in both cases.

The fact that the profile of PAH found in the moss clone "Mosssphere" was the same as in the total deposition allows to establish relations between the different PAH characteristic of different source emissions. Therefore, moss clone spheres can simulate quite well the distribution obtained with the conventional device and give us information about the possible sources of these pollutants.

### Example 3 - Use of the devices for heavy metal sensing

In this experiment, the performance of bulk deposimeters and *Mossspheres* with 3 g or moss clone with standardized shape, mesh and moss content (Figure 4, left) were compared, exposing both in 10 sites (6 in Italy and 4 in Spain) for 6 weeks.

The exposure of the moss clone bags was performed in the traditional way, without covering the *Mosssphere* at an height of 4 meters above ground. At the end of the exposure period, the *Mossspheres* were collected and stored in zip-lock closure plastic bags. Each plastic bag was opened in the lab (including non-exposed bags labelled as blank), the net containing the moss clone was removed and all samples were dried at 40 °C for 8 h, milled and sent to the laboratory for analysis. Metalloid and metal analysis of moss samples were conducted by inductively coupled plasma mass spectrometry (ICP-MS). The main meteorological parameters (air temperature, wind speed and direction, atmospheric pressure and precipitations) were recorded for all the exposure periods.

### Results

The concentration of some of the 19 elements measured were below the detection limit. For this reason the comparisons between the values of total deposition flux for metals in bulk deposimeters and the concentration of metals in mosses was only possible for nine elements: Al, Ba, Cu, Fe, Pb, Ni, Sr, V and Zn.

The results from statistical analysis (Spearman's rank correlation test) showed a good correlation for Al, Ba, Cu and Sr (p-value< 0.05), a borderline situation for Pb (p-value=0.06), and a weak correlation for Fe, Ni, V and Zn.

## Claims

1. Passive contaminant sensor device comprising an axenic devitalized moss clone.

2. Passive contaminant sensor device in accordance with claim 1 wherein the moss clone is obtained from *Sphagnum* mosses (*Sphagnum* sp.), sheet mosses (*Hypnum* sp.), *Pseudoscleropodium purum* or *Brachythecium rutabulum.*

3. Passive contaminant sensor device in accordance with claim 2 wherein the moss is a *Sphagnum* moss.

4. Passive contaminant sensor device in accordance with claim 3 wherein the *Sphagnum* moss is *Sphagnum palustre.*

5. Passive contaminant sensor device in accordance with any of claims 1 to 4 wherein the BET specific surface area of the clone exceeds the specific surface area of the natural moss from which the clone was obtained, by at least 50 %.

6. Passive contaminant sensor device in accordance with any of claims 1 to 5 wherein the Al-content of the clone, based on dry weight, is at least 50 % below the Al-content of the natural moss from which the clone was obtained.

7. Passive contaminant sensor device in accordance with any of claims 1 to 6 wherein the Fe-content of the clone, based on dry weight, is at least 50 % below the Fe-content of the natural moss from which the clone was obtained.

8. Passive contaminant sensor device in accordance with any of claims 1 to 7 wherein the content of the clone of at least one of the elements derived from Na, Mg, Ca, Ti, V, Mn, Co, Ni, Cu, Zn, Ga, Ge, A, Rb, Sr, Zr, Nb, Ag, Cd, Cs, Ba, La, Ce, Pr, Nd, Sm, Eu, Gd, Dy and Ho is at least 50 % below the content of the respective element in the natural moss form which the clone was obtained.

9. Passive contaminant sensor device in accordance with any of claims 1 to 8 for the sensing of air pollutants.

10. Passive contaminant sensor device in accordance with claim 9 wherein the air pollutants comprise of or consist of polycyclic aromatic hydrocarbons or heavy metals or their compounds.

11. Use of an axenic devitalized moss clone as passive contaminant sensor.

12. Use in accordance with claim 11 wherein the moss clone is obtained from *Sphagnum* mosses (*Sphagnum* sp.), sheet mosses (*Hypnum* sp.), *Pseudoscleropodium purum* or *Brachythecium rutabulum,* preferably a *Sphagnum* moss and even more preferably *Sphagnum palustre.*

13. Use in accordance with claims 11 or 12 wherein the contaminants are air pollutants.

14. Use in accordance with any of claims 11 to 13 wherein the air pollutants comprise of or consist of polycyclic aromatic hydrocarbons or heavy metals or their compounds or mixtures thereof.
